# EUROPÄISCHE PATENTSCHRIFT

(11) **EP 1 730 126 B1**
(45) Veröffentlichungstag und Bekanntmachung des Hinweises auf die Patenterteilung: **11.01.2012**
(21) Anmeldenummer: 05715856.0
(22) Anmeldetag: 09.03.2005
(51) Int. Cl.: C07D 265/36, A61K 31/536, A61P 11/00

(54) **NEUE BENZOXAZINONDERIVATE ALS LANGWIRKSAME BETAMIMETIKA ZUR BEHANDLUNG VON ATEMWEGSERKRANKUNGEN**
NOVEL BENZOXAZINONE DERIVATIVES USED AS LONG-ACTING BETAMIMETICS FOR TREATING RESPIRATORY ILLNESSES
NOUVEAUX DERIVES DE BENZOXAZINONE COMME BETA-MIMETIQUES DE LONGUE DUREE ET LEUR UTILISATION POUR LE TRAITEMENT DES MALADIES DES VOIES RESPIRATOIRES

(30) Priorität: 17.03.2004 EP 04006348
(43) Veröffentlichungstag der Anmeldung: 13.12.2006
(73) Patentinhaber: BOEHRINGER INGELHEIM INTERNATIONAL GMBH, 55216 Ingelheim (DE); Boehringer Ingelheim Pharma GmbH & Co. KG, 55216 Ingelheim (DE)
(72) Erfinder: BOUYSSOU, Thierry, 88447 Birkenhard (DE); HOENKE, Christoph, 55218 Ingelheim (DE); KONETZKI, Ingo, 88447 Warthausen (DE); SCHNAPP, Andreas, 88400 Biberach (DE)
(74) Vertreter: Hammann, Heinz
(86) Internationale Anmeldenummer: PCT/EP2005/002462
(87) Internationale Veröffentlichungsnummer: WO 2005/092870

(56) Entgegenhaltungen:
- WO-A-01/83462
- WO-A-03/087097
- US-A- 4 460 581

## Beschreibung

Die vorliegende Erfindung betrifft die Verbindungen der allgemeinen Formel **1** 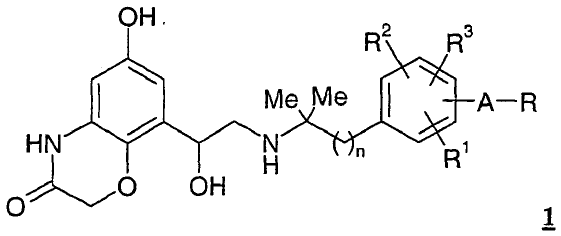 worin die Reste R, R¹, R², R³ und A die in den Ansprüchen und in der Beschreibung genannten Bedeutungen haben können, Verfahren zu deren Herstellung sowie deren Verwendung als Arzneimittel, insbesondere zur Behandlung von entzündlichen und obstruktiven Atemwegserkrankungen.

### Hintergrund der Erfindung

Betamimetika (ß-adrenerge Substanzen) sind aus dem Stand der Technik bekannt. Beispielsweise sei diesbezüglich auf die Offenbarung der US 4,460,581 verwiesen, die Betamimetika zur Therapie unterschiedlichster Erkrankungen vorschlägt.

Zur medikamentösen Therapie von Erkrankungen ist es häufig wünschenswert, Arzneimittel mit einer längeren Wirkungsdauer bereitzustellen. Hierdurch kann in der Regel gewährleistet werden, daß die zur Erzielung des therapeutischen Effekts erforderliche Konzentration des Wirkstoffs im Organismus über einen längeren Zeitraum gegeben ist, ohne eine allzu häufige, wiederholte Gabe des Arzneimittels durchführen zu müssen. Die Applikation eines Wirkstoffs in längeren zeitlichen Abständen trägt im übrigen in hohem Maße zum Wohlbefinden des Patienten bei.

Es ist daher Aufgabe der vorliegenden Erfindung, Betamimetika bereitzustellen, die einerseits bei der Therapie von entzündlichen und obstruktiven Atemwegserkrankungen, besonders bevorzugt bei der Therapie des Asthma oder der COPD einen therapeutischen Nutzen entfalten und darüberhinaus durch eine längere Wirkdauer gekennzeichnet sind und somit zur Herstellung von Arzneimitteln mit längerer Wirksamkeit Verwendung finden können. Neben den vorstehend genannten Aufgaben ist es ferner Ziel der vorliegenden Erfindung, solche Betamimetika bereitzustellen, die nicht nur außerordentlich potent, sondern ferner durch ein hohes Maß an Selektivität gegenüber dem β₂-Adrenozeptor gekennzeichnet sind.

### Detaillierte Beschreibung der Erfindung

Überraschenderweise wurde gefunden, daß die vorstehend genannten Aufgaben durch Verbindungen der allgemeinen Formel **1** gelöst werden.

Dementsprechend betrifft die vorliegende Erfindung Verbindungen der allgemeinen Formel **1** 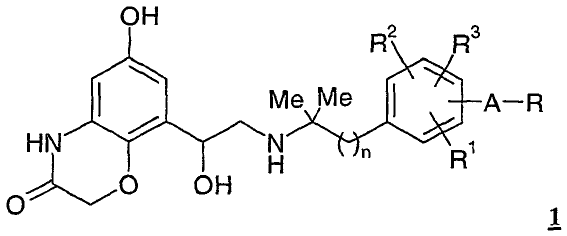 worin
- n: 1 oder 2, bevorzugt 1;
- A: Sauerstoff oder eine Einfachbindung;
- R: -C₁-C₆-Alkyl, dass ein- oder mehrfach durch ein oder mehrere Halogenatome substituiert ist;
- R¹, R² und R³: gleich oder verschieden, Wasserstoff, C₁-C₆-Alkyl, Halogen-C₁-C₆-alkylen, OH, HO-C₁₋₆-alkylen, -O-C₁-C₆-Alkyl, C₆-C₁₀-Aryl, C₆-C₁₀-Aryl-C₁-C₄-alkylen, C₆-C₁₀-Aryl-C₁-C₆-alkylen-O-, -COOH, -COOC₁-C₆-alkyl, -O-C₁-C₆-alkylen-COOH, -O-C₁-C₆-alkylen-COOC₁-C₆-alkyl, -NHSO₃H, -NHSO₂-C₁-C₆-alkyl, CN, NH₂, -NH-C₁-C₆-Alkyl, -N(C₁-C₆-Alkyl)₂ ,NO₂, -S-C₁-C₆-Alkyl, -SO₂-C₁-C₆-Alkyl, -SO-C₁-C₆-Alkyl, -O(CO)C₁-C₆-Alkyl, -COC₁-C₆-Alkyl, -NHCOC₁-C₆-Alkyl oder Halogen bedeuten.

Bevorzugt sind Verbindungen der allgemeinen Formel **1**, worin
- n: 1 oder 2, bevorzugt 1,
- A: Sauerstoff oder eine Einfachbindung;
- R: C₁-C₄-Alkyl, dass ein- oder mehrfach, bevorzugt ein- bis dreifach, durch ein oder mehrere Halogenatome, bevorzugt durch Fluor oder Chlor, substituiert ist;
- R¹, R² und R³: gleich oder verschieden, Wasserstoff, C₁-C₄-Alkyl, Halogen-C₁-C₄-alkylen, OH, HO-C₁-C₄-alkylen, -O-C₁-C₄-Alkyl, Phenyl, Phenyl-C₁-C₄-alkylen, Phenyl-C₁-C₄-alkylen-O-, -COOH, -COOC₁-C₄-alkyl, -O-C₁-C₄-alkylen-COOH, -O-C₁-C₄-alkylen-COOC₁-C₄-alkyl, -NHSO₂-C₁-C₄-alkyl, CN, NH₂, -NH-C₁-C₄-Alkyl, -N(C₁-C₄-Alkyl)₂, NO₂, -S-C₁-C₄-Alkyl, -SO₂-C₁-C₄-Alkyl, -SO-C₁-C₄-Alkyl, -O(CO)C₁-C₄-Alkyl, -COC₁-C₄-Alkyl, -NHCOC₁-C₄-Akyl oder Halogen bedeuten.

Bevorzugt sind ferner Verbindungen der allgemeinen Formel **1**, worin
- n: 1 oder 2, bevorzugt 1,
- A: Sauerstoff oder eine Einfachbindung;
- R: ein Methl- oder Ethylrest, der ein-, zwei- oder dreifach durch ein oder mehrere Halogenatome ausgewählt aus Fluor oder Chlor substituiert ist;
- R¹ und R²: gleich oder verschieden, bevorzugt gleich, Wasserstoff, Halogen, C₁-C₄-Alkyl oder -O-C₁-C₄-Alkyl;
- R³: Wasserstoff, C₁-C₄-Alkyl, OH, Halogen, -O-C₁-C₄-Alkyl, Halogen-C₁-C₄-alkylen, -O-C₁-C₄-Alkylen-COOH, -O-C₁-C₄-Alkylen-CO-O-C₁-C₄-Alkyl bedeuten.

Bevorzugt sind Verbindungen der allgemeinen Formel **1**, worin
- n: 1;
- A: Sauerstoff oder eine Einfachbindung, bevorzugt eine Einfachbindung;
- R: -CH₂-CH₂Cl, -CH₂CCl₃, -CHCl-CCl₃, -CCl₃, -CH₂Cl, -CHCl₂, -CH₂CF₃, -CHF-CF₃, -CF₃, -CH₂F, -CH₂-CH₂F oder -CHF₂;
- R¹ und R²: gleich oder verschieden, bevorzugt gleich, Wasserstoff, Fluor, Chlor, Methyl oder Methoxy;
- R³: Wasserstoff, C₁-C₄-Alkyl, OH, Fluor, Chlor, Brom, -O-C₁-C₄-Alkyl, -CF₃, -O-C₁-C₄-Alkylen-COOH, -O-C₁-C₄-Alkylen-CO-O-C₁-C₄-Alkyl bedeuten.

Besonders bevorzugt sind die Verbindungen der allgemeinen Formel **1**, worin
- n: 1;
- A: Sauerstoff oder eine Einfachbindung, bevorzugt eine Einfachbindung;
- R: -CH₂CF₃, -CHF-CF₃, -CF₃, -CH₂F, -CH₂-CH₂F oder -CHF₂;
- R¹ und R²: gleich oder verschieden, bevorzugt gleich, Wasserstoff, Fluor, Chlor, Methyl oder Ethyl;
- R³: Wasserstoff, Fluor, Chlor, Methyl, Ethyl, OH, Methoxy, Ethoxy, -CF₃, -O-CH₂-COOH, -O-CH₂-CO0Methyl oder -O-CH₂-COOEthyl, bedeuten.

Besonders bevorzugt sind ferner die vorstehend genannten Verbindungen der allgemeinen Formel **1**, worin R¹ und R² Wasserstoff und die Gruppen A, R und R³ die vorstehend genannten Bedeutungen haben können.

Besonders bevorzugt sind ferner die vorstehend genannten Verbindungen der allgemeinen Formel **1**, worin A für eine Einfachbindung steht und die Gruppen R, R¹, R² und R³ die vorstehend genannten Bedeutungen haben können.

Besonders bevorzugt sind ferner die vorstehend genannten Verbindungen der allgemeinen Formel **1**, worin R³ Wasserstoff, Fluor, Chlor, Methyl, Ethyl, OH, Methoxy oder Ethoxy, bevorzugt Wasserstoff, Fluor oder Chlor, besonders bevorzugt Wasserstoff und die Gruppen A, R, R¹ und R² die vorstehend genannten Bedeutungen haben können.

Besonders bevorzugt sind ferner die vorstehend genannten Verbindungen der allgemeinen Formel **1**, worin R -CF₃, -CH₂F oder -CHF₂, bevorzugt -CF₃ bedeutet und die Gruppen A, R¹, R² und R³ die vorstehend genannten Bedeutungen haben können.
Besonders bevorzugt sind ferner Verbindungen der allgemeinen Formel **1**, die ausgewählt sind aus der Gruppe bestehend aus
8-{2-[2-(4-Fluor-3-trifluormethyl-phenyl)-1,1-dimethyl-ethylamino]-1-hydroxy-ethyl}-6-hydroxy-4H-benzo[1,4]oxazin-3-on;
8-{2-[1,1-Dimethyl-2-(4-trifluoromethyl-phenyl)-ethylamino]-1-hydroxy-ethyl}-6-hydroxy-4H-benzo[1,4]oxazin-3-on;
8-{2-[1,1-Dimethyl-2-(3-trifluomethyl-phenyl)-ethylamino]-1-hydroxy-ethyl}-6-hydroxy-4H-benzo[1,4]oxazin-3-on;
8-{2-[1,1-Dimethyl-2-(4-trifluormethoxy-phenyl)-ethylamino]-1-hydroxy-ethyl}-6-hydroxy-4H-benzo[1,4]oxazin-3-on;
8-{2-[2-(4-Chlor-3-trifluormethyl-phenyl)-1,1-dimethyl-ethylamin]-1-hydroxy-ethyl}-6-hydroxy-4H-benzo[1,4]oxazin-3-on;
8-{2-[1,1-Dimethyl-2-(2-trifluormethyl-phenyl)-ethylamino]-1-hydroxy-ethyl}-6-hydroxy-4H-benzo[1,4]oxazin-3-on;
8-{2-[2-(3,5-Bis-trifluormethyl-phenyl)-1,1-dimethyl-ethylamino]-1-hydroxy-ethyl}-6-hydroxy-4H-benzo[1,4]oxazin-3-on.

Ein weiterer Aspekt der vorliegenden Erfindung betrifft die vorstehend genannten Verbindungen der Formel **1** in Form der einzelnen optischen Isomeren, Mischungen der einzelnen Enantiomeren oder Racemate. Besonders bevorzugt sind dabei Verbindungen der Formel **1** in Form der enantiomerenreinen Verbindungen, wobei die R-Enantiomere der Verbindungen der Formel **1** erfindungsgemäß von herausragender Bedeutung sind. Verfahren zur Auftrennung von Racematen in die jeweiligen Enantiomere sind im Stand der Technik bekannt und können zur Darstellung der enantiomerenreinen R- bzw. S-Enantiomere der Verbindungen der Formel **1** in analoger Art und Weise zur Anwendung gelangen.

Ein weiterer Aspekt der vorliegenden Erfindung betrifft die vorstehend genannten Verbindungen der Formel **1** in Form ihrer Säureadditionssalze mit pharmakologisch unbedenklichen Säuren sowie gegebenenfalls in Form der Solvate und/oder Hydrate. Unter Säureadditionssalzen mit pharmakologisch unbedenklichen Säuren werden dabei beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Von den vorstehend genannten Säureadditionssalzen sind die Salze der Chlorwasserstoffsäure, der Methansulfonsäure, der Benzoesäure und der Essigsäure erfindungsgemäß besonders bevorzugt.

Ein weiterer Aspekt der vorliegenden Erfindung betrifft die vorstehend genannten neuen Verbindungen der Formel **1** zur Verwendung als Arzneimittel. Ferner betrifft die vorliegende Erfindung die Verwendung der vorstehend genannten neuen Verbindungen der Formel **1** zur Herstellung eines Arzneimittels zur Behandlung von entzündlichen und obstruktiven Atemwegserkrankungen, besonders bevorzugt zur Behandlung des Asthma oder der COPD. Ferner betrifft die vorliegende Erfindung die Verwendung der vorstehend genannten neuen Verbindungen der Formel **1** zur Herstellung eines Arzneimittles zur einode zweimal täglichen Behandlung von entzündlichen und obstruktiven Atemwegserkrankungen, besonders bevorzugt des Asthma oder der COPD.

Ferner betrifft die vorliegende Erfindung ein Verfahren zur Behandlung von entzündlichen und obstruktiven Atemwegserkrankungen, besonders bevorzugt zur Behandlung des Asthma oder der COPD., dadurch gekennzeichnet, daß eine oder mehrere der vorstehend genannten Verbindungen der allgemeinen Formel **1** in therapeutisch wirksamen Mengen appliziert werden.

Zur erfindungsgemäßen Verwendung können die Verbindungen der allgemeinen Formel **1** gegebenenfalls in Form der einzelnen optischen Isomeren, Mischungen der einzelnen Enantiomeren oder Racemate zur Anwendung gelangen. Werden die Verbindungen in enantiomerenreiner Form eingesetzt, werden bevorzugt die R-Enantiomere verwendet.

Als Alkylgruppen werden, soweit nicht anders angegeben, verzweigte und unverzweigte Alkylgruppen mit 1 bis 4 Kohlenstoffatomen bezeichnet. Beispielsweise werden genannt: Methyl, Ethyl, Propyl oder Butyl. Zur Bezeichnung der Gruppen Methyl, Ethyl, Propyl oder auch Butyl werden gegebenenfalls auch die Abkürzungen Me, Et, Prop oder Bu verwendet. Sofern nicht anders beschrieben, umfassen die Definitionen Propyl und Butyl alle denkbaren isomeren Formen der jeweiligen Reste. So umfaßt beispielsweise Propyl n-Propyl und iso-Propyl, Butyl umfaßt iso-Butyl, sec. Butyl und tert.-Butyl etc.

Als Alkylengruppen werden, soweit nicht anders angegeben, verzweigte und unverzweigte zweibindige Alkylbrücken mit 1 bis 4 Kohlenstoffatomen bezeichnet. Beispielsweise werden genannt: Methylen, Ethylen, n-Propylen oder n-Butylen.

Als Alkyloxygruppen (oder auch -O-Alkylgruppen) werden, soweit nicht anders angegeben, verzweigte und unverzweigte Alkylgruppen mit 1 bis 4 Kohlenstoffatomen bezeichnet, die über ein Sauerstoffatom verknüpft sind. Beispielsweise werden genannt: Methyloxy, Ethyloxy, Propyloxy oder Butyloxy. Zur Bezeichnung der Gruppen Methyloxy, Ethyloxy, Propyloxy oder auch Butyloxy werden gegebenenfalls auch die Abkürzungen MeO-, EtO-, PropO- oder BuO- verwendet. Sofern nicht anders beschrieben, umfassen die Definitionen Propyloxy und Butyloxy alle denkbaren isomeren Formen der jeweiligen Reste. So umfaßt beispielsweise Propyloxy n-Propyloxy und iso-Propyloxy, Butyloxy umfaßt iso-Butyloxy, sec. Butyloxy und tert.-Butyloxy etc. Gegebenenfalls wird im Rahmen der vorliegenden Erfindung statt der Bezeichnung Alkyloxy auch die Bezeichnung Alkoxy verwendet. Zur Bezeichnung der Gruppen Methyloxy, Ethyloxy, Propyloxy oder auch Butyloxy gelangen dementsprechend gegebenenfalls auch die Ausdrücke Methoxy, Ethoxy, Propoxy oder Butoxy zur Anwendung.

Halogen steht im Rahmen der vorliegenden Erfindung für Fluor, Chlor, Brom oder Jod. Sofern nicht gegenteilig angegeben, gelten Fluor, Chlor und Brom als bevorzugte Halogene, wobei Fluor besonders bevorzugt ist.

Aryl steht, soweit nicht anders angegeben, für ein aromatisches Ringsystem mit 6 bis 10 Kohlenstoffatomen. Bevorzugte Arylgruppen sind im Rahmen der vorliegenden Erfindung Phenyl und Naphthyl.

Halogen-alkylen steht, soweit nicht anders angegeben, für verzweigte oder unverzweigte Alkylgruppen, die wenigstens durch ein, gegebenenfalls auch mehrere Halogenatome substituiert sind. HO-alkylen steht, soweit nicht anders angegeben, für verzweigte oder unverzweigte Alkylgruppen, die durch OH substituiert sind. Aryl-alkylen steht, soweit nicht anders angegeben, für verzweigte oder unverzweigte Alkylgruppen, die durch einen Arylrest substituiert sind. Beispiele für erfindungsgemäß bevorzugte Aryl-alkylen-Gruppen sind Benzyl, Phenylethyl, Naphthylmethyl, Naphthylethyl, wobei Benzyl und Phneylethyl erfindungsgemäß besonders bevorzugt sind.

Die Herstellung der erfindungsgemäßen Verbindungen kann in Analogie zu im Stand der Technik bereits bekannten Vorgehensweisen erfolgen. Geeignete Herstellverfahren sind beispielsweise aus der US 4-460581 bekannt, auf die an dieser Stelle vollinhaltlich Bezug genommen wird.

Die nachstehend beschriebenen Beispiele dienen der weitergehenden Illustration von aus dem Stand der Technik bekannten Verbindungen, die entsprechend der vorliegenden Erfindung überraschenderweise zur Therapie von COPD Verwendung finden können.

### Beispiel 1: 8-{2-[2-(4-Fluor-3-trifluormethyl-phenyl)-1,1-dimethyl-ethylamino]-1-hydroxy-ethyl}-6-hydroxy-4H-benzo [1,4]oxazin-3-on

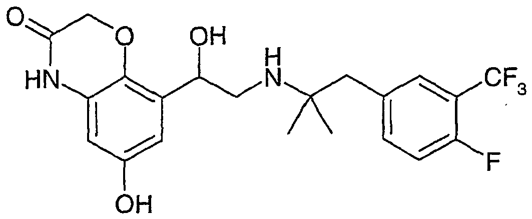

### a) 1-(4-Fluor-3-trifluormethyl-phenyl)-2-methyl-propan-1-ol

Aus 10 g (40 mmol) 5-Bromo-2-fluorbenzotrifluorid, gelöst in 50 mL Diethylether, und 0.97 g (40 mmol) Magnesium wird ein Grignard hergestellt. Anschließend tropft man eine Lösung von 3.63 mL (40 mmol) Isobutyraldehyd und 30 mL Diethylether bei Raumtemperatur zu und läßt dann über Nacht rühren. Die Reaktionsmischung wird auf Eiswasser gegossen, mit 30 mL 20%iger Schwefelsäure versetzt und mit Diethylether extrahiert. Die vereinigten organischen Phasen werden nacheinander mit Natriumhydrogencarbonat-Lösung, Wasser und Natriumchlorid-Lösung gewaschen, mit Natriumsulfat getrocknet und eingeengt. Die chromatographische Reinigung des Rückstands liefert die Zielverbindung in Form eines braunen Öls. Ausbeute: 4.3 g (46%); Massenspektroskopie: [M]⁺ = 236.

### b) 1-Fluor-4-(2-methyl-propenyl)-2-trifluormethyl-benzen

4.3 g (18.2 mmol) 1-(4-Fluor-3-trifluormethyl-phenyl)-2-methyl-propan-1-ol und 1.0 g (5.3 mmol) p-Toluensulfonsäure-Monohydrat in 100 mL Toluol werden 3 Stunden am Wasserabscheider unter Rückfluß gekocht. Anschließend wird die Reaktionsmischung mit Wasser versetzt und mit 1 N Natriumhydroxid-Lösung alkalisch gestellt. Nach Abtrennung der organischen Phase wird diese mit Wasser gewaschen, mit Natriumsulfat getrocknet und eingeengt. Das zurückbleibende Öl wird direkt weiter umgesetzt. Ausbeute: 3.47 g; Massenspektroskopie: [M]⁺ = 218.

### c) N-[2-(4-Fluor-3-trifluormethyl-phenyl)-1,1-dimethyl-ethyl]-formamid

Zu 1.0 g (20.4 mmol) Natriumcyanid in 15 mL Eisessig wird eine Lösung aus 30 mL konz. Schwefelsäure und 15 mL Eisessig getropft, wobei die Temperatur auf ca. 30°C ansteigt. Anschließend werden 3.4 g (15.6 mmol) 1-Fluor-4-(2-methyl-propenyl)-2-trifluormethylbenzen, gelöst in 15 mL Eisessig, zugegeben und es wird 1 Stunde bei 50-60°C gerührt. Nach Abkühlung auf Raumtemperatur wird der Ansatz auf Eiswasser gegossen und mit Natronlauge alkalisch gestellt. Es wird mit Diethylether extrahiert und die organischen Phasen werden mit Wasser gewaschen, mit Natriumsulfat getrocknet und eingeengt. Es werden 3.64 g eines braunen Öls erhalten. Massenspektroskopie: [M+H]⁺ = 264.

### d) 2-(4-Fluor-3-trifluormethyl-phenyl)-1,1-dimethyl-ethylamin

3.30 g (12.5 mmol) N-[2-(4-Fluor-3-trifluormethyl-phenyl)-1,1-dimethyl-ethyl]-formamid werden mit 25 mL Wasser und 25 mL konz. Salzsäure versetzt und 2 Stunden unter Rückfluß gekocht. Das Reaktionsgemisch wird mit Wasser verdünnt, mit Kaliumcarbonat-Lösung alkalisch gestellt und mit Dichlormethan extrahiert. Die vereinigten organischen Phasen werden mit Natriumsulfat getrocknet und vom Lösungsmittel befreit. Braunes Öl. Ausbeute: 2.8 g (95%); Massenspektroskopie: [M+H]⁺ = 236.

### e) 8-{2-[2-(4-Fluor-3-trifluormethyl-phenyl)-1,1-dimethyl-ethylamino]-1-hydroxy-ethyl}-6-hydroxy-4H-benzo[1,4]oxazin-3-on

357 mg (1 mmol) 6-Benzyloxy-8-(2-ethoxy-2-hydroxy-acetyl)-4H-benzo[1,4]oxazin-3-on und 235 mg (1 mmol) 2-(4-Fluor-3-trifluormethyl-phenyl)-1,1-dimethyl-ethylamin werden in Analogie zu dem für Beispiel 2c) beschriebenen Verfahren umgesetzt. Beigefarbener Feststoff. Ausbeute: 265 mg (48%, Trifluoracetat); Massenspektroskopie: [M+H]⁺ = 443.

### Beispiel 2: 8- {2-[1,1-Dimethyl-2-(4-trifluormethyl-phenyl)-ethylamino]-1-hydroxy-ethyl}-6-hydroxy-4H-benzo[1,4]oxazin-3-on

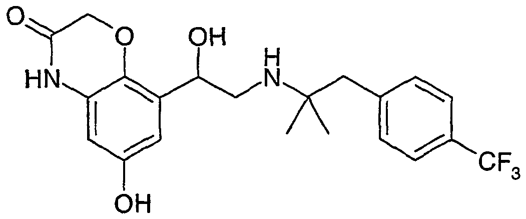

### a) N-[1,1-Dimethyl-2-(4-trifluormethyl-phenyl)-ethyl]-formamid

4.2 g (19 mmol) 2-Methyl-1-(4-trifluormethyl-phenyl)-propanol-2-ol, erhalten aus der Umsetzung von (4-Trifluormethyl-phenyl)-essigsäureethylester mit Methylmagnesiumbromid, werden nach der Arbeitsvorschrift für Beispiel 1c) umgesetzt und aufgearbeitet. Ausbeute: 4.6 g (98%).

### b) 1,1-Dimethyl-2-(4-trifluormethyl-phenyl)-ethylamin

Erhalten aus 4.6 g (19 mmol) N-[1,1-Dimethyl-2-(4-trifluormethyl-phenyl)-ethyl]-formamid in Analogie zur Vorschrift für Beispiel 1d). Ausbeute: 3.8 g (93%); Massenspektroskopie: [M+H]⁺ = 218.

### c) 8-{2-[1,1-Dimethyl-2-(4-trifluormethyl-phenyl)-ethylaminol-1-hydroxy-ethyl}-6-hydroxy-4H-benzo [1,4]oxazin-3-on

357 mg (1 mmol) 6-Benzyloxy-8-(2-ethoxy-2-hydroxy-acetyl)-4H-benzo[1,4]oxazin-3-on und 217 mg (1 mmol) 1,1-Dimethyl-2-(4-trifluormethyl-phenyl)-ethylamin werden 30 Minuten in 5 mL Tetrahydrofuran bei Raumtemperatur gerührt. Man kühlt auf 0°C ab und tropft unter Argonatmosphäre 1.5 mL einer 2 molaren Lösung von Lithiumborhydrid in Tetrahydrofuran zu. Es wird 30 min bei Raumtemperatur gerührt, mit 10 mL Dichlormethan und 3 mL Wasser versetzt, eine weitere Stunde gerührt und dann über Kieselgur filtriert. Man eluiert mit Dichlormethan und destilliert die Lösungsmittel ab. Der Rückstand wird in Methanol gelöst und mit Palladium auf Kohle (10%ig) als Katalysator bei 2.5 bar und Raumtemperatur hydriert. Der Katalysator wird abgetrennt und das Rohprodukt chromatographisch (Reverse Phase, Acetonitril/Wasser-Gradient mit 0.1% Trifluoressigsäure) gereinigt. Beigefarbener Feststoff. Ausbeute: 212 mg (39%, Trifluoracetat); Massenspektroskopie: [M+H]⁺ = 425.

### Beispiel 3: 8-{2-[1,1-Dimethyl-2-(3-trifluomethyl-phenyl)-ethylamino]-1-hydroxy-ethyl}-6-hydroxy-4H-benzo[1,4]oxazin-3-on

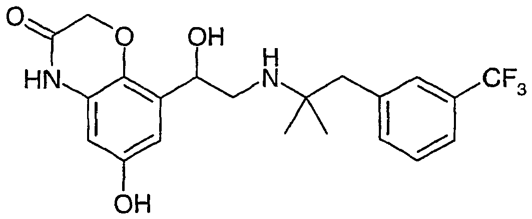

### a) 2-Methyl-1-(3-trifluormethyl-phenyl)-propan-2-ol

90 mL einer 3 molaren Lösung von Methylmagnesiumbromid in Diethylether werden mit 300 mL THF verdünnt und auf -50°C abgekühlt. Bei dieser Temperatur werden 20 g 1-(3-Trifluormethyl-phenyl)-propane-2-on, gelöst in 100 mL THF, zugetropft. Nach beendeter Zugabe wird das Reaktionsgemisch unter Erwärmung auf Raumtemperatur über Nacht gerührt. Es wird mit Ammoniumchlorid-Lösung versetzt und mit Diethylether extrahiert. Die vereinigten organischen Phasen werden mit Natriumchlorid-Lösung gewaschen, mit Natriumsulfat getrocknet und eingeengt. Der verbleibende Rückstand wird chromatographisch gereinigt. Ausbeute: 9.5 g (44%).

### b) N-[1,1-Dimethyl-2-(3-trifluormethyl-phenyl)-ethyl]-formamid

9.5 g (44 mmol) 2-Methyl-1-(3-trifluormethyl-phenyl)-propan-2-ol werden in einer Ritter-Reaktion analog zur Vorschrift für Beispiel 1c) umgesetzt. Gelbes Öl. Ausbeute: 9.8 g (92%); Massenspektroskopie: [M+H]⁺ = 246.

### c) 1,1-Dimethyl-2-(3-trifluormethyl-phenyl)-ethylamin

9.8 g (40 mmol) N-[1,1-Dimethyl-2-(3-trifluormethyl-phenyl)-ethyl]-formamid werden in 110 mL Ethanol gelöst, mit 200 ml konz. Salzsäure versetzt und über Nacht refluxiert. Die Reaktionsmischung wird auf Eiswasser gegeben, mit Natriumhydroxid alkalisch gestellt und mit tert.-Butylmethylether extrahiert. Die organischen Phasen werden mit Wasser gewaschen, getrocknet und eingeengt. Anschließend wird der Rückstand chromatographisch gereinigt. Ausbeute: 1.9 g (22%); Massenspektroskopie: [M+H]⁺ = 218.

### d) 8-{2-[1,1-Dimethyl-2-(3-trifluormethyl-phenyl)-ethylamino]-1-hydroxy-ethyl}-6-hydroxy-4H-benzo[1,4]oxazin-3-on

357 mg (1 mmol) 6-Benzyloxy-8-(2-ethoxy-2-hydroxy-acetyl)-4H-benzo[1,4]oxazin-3-on und 217 mg (1 mmol) 1,1-Dimethyl-2-(3-trifluormethyl-phenyl)-ethylamin werden 3 Stunden in 5 mL Ethanol bei 65°C gerührt. Man kühlt auf Raumtemperatur ab, gibt 113 mg (3 mmol) Natriumborhydrid hinzu und läßt über Nacht rühren. Es wird mit 2 mL Wasser versetzt, 30 Minuten gerührt und dann mit 10 mL Dichlormethan verdünnt. Die Lösung wird über Kieselgur filtriert und mit Dichlormethan nachgewaschen. Das Eluat wird vom Lösungsmittel befreit und der Rückstand in 5 mL Ethanol gelöst und mit Palladium auf Kohle (10%ig) als Katalysator bei 2.5 bar und Raumtemperatur hydriert. Anschließend wird der Katalysator abgetrennt und das Rohprodukt chromatographisch (Reverse Phase, Acetonitril/Wasser-Gradient mit 0.1% Trifluoressigsäure) gereinigt. Beigefarbener Feststoff. Ausbeute: 169 mg (31%, Trifluoracetat); Massenspektroskopie: [M+H]⁺ = 425.

### Beispiel 4: 8-{2-[1,1-Dimethyl-2-(4-trifluormethoxy-phenyl)-ethylamino]-1-hydroxyethyl}-6-hydroxy-4H-benzo[1,4]oxazin-3-on

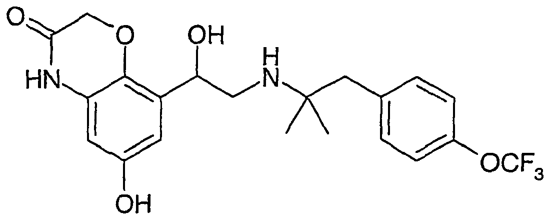

### a) 2-Methyl-1-(4-trifluormethoxy-phenyl)-propan-1-ol

Die Darstellung der Zielverbindung erfolgt in Analogie zu dem für Beispiel 1a) beschriebenen Verfahren. Aus 23.7 g (96 mmol) 1-Brom-4-(trifluormethoxy)-benzol und 2.35 g (97 mmol) Magnesium wird zunächst ein Grignard hergestellt, der dann mit 7.3 g (100 mol) Isobutyraldehyd umgesetzt wird. Nach Aufarbeitung und säulenchromatographischer Reinigung werden 14.8 g eines braunen Öls erhalten, das direkt weiter umgesetzt wird.

### b) 1-(2-Methyl-propenyl)-4-trifluormethoxy-benzen

14.8 g (63 mmol) 2-Methyl-1-(4-trifluormethoxy-phenyl)-propan-1-ol werden wie in der Vorschrift für Beispiel 1b) beschrieben umgesetzt und aufgearbeitet. Ausbeute: 9.8 g (72%); Massenspektroskopie: [M]⁺ = 216.

### c) N-[1,1-Dimethyl-2-(4-trifluomethoxy-phenyl)-ethyl]-formamid

Eine Ritter-Reaktion mit 9.8 g (45 mmol) 1-(2-Methyl-propenyl)-4-trifluormethoxy-benzen, durchgeführt wie für Beispiel 1c) beschrieben, liefert 7.8 g leicht verunreinigtes Produkt. Massenspektroskopie: [M+H]⁺ = 262.

### d) 1,1-Dimethyl-2-(4-trifluormethoxy-phenyl)-ethylamin

7.8 g (30 mmol) N-[1,1-Dimethyl-2-(4-trifluomethoxy-phenyl)-ethyl]-formamid und 3.5 g Kaliumhydroxid werden über Nacht bei 140°C in 30 mL Ethylenglykol gerührt. Nach Abkühlung auf Raumtemperatur wird die Reaktionsmischung mit Wasser verdünnt und mit Dichlormethan extrahiert. Die vereinigten organischen Phasen werden mit Wasser gewaschen, mit Natriumsulfat getrocknet und eingeengt. Das zurückbleibende Öl wird über eine kurze Kieselgelsäule (Dichlormethan/Methanol/Ammoniak = 90/10/1) gereinigt. Ausbeute: 4.0 g (57%); Massenspektroskopie: [M+H]⁺ = 234.

### e) 8-{2-[1,1-Dimethyl-2-(4-trifluormethoxy-phenyl)-ethylamino]-1-hydroxy-ethyl}-6-hydroxy-4H-benzo [1,4]oxazin-3-on

Die Zielverbindung wird aus der Umsetzung von 357 mg (1 mmol) 6-Benzyloxy-8-(2-ethoxy-2-hydroxy-acetyl)-4H-benzo[1,4]oxazin-3-on mit 233 mg (1 mmol) 1,1-Dimethyl-2-(4-trifluormethoxy-phenyl)-ethylamin nach dem für Beispiel 2c) beschriebenen Verfahren erhalten. Beigefarbener Feststoff. Ausbeute: 279 mg (50%, Trifluoracetat); Massenspektroskopie: [M+H]⁺ = 441.

### Beispiel 5: 8-{2-[2-(4-Chlor-3-trifluormethyl-phenyl)-1,1-dimethyl-ethylamin]-1-hydroxyethyl}-6-hydroxy-4H-benzo[1,4]oxazin-3-on

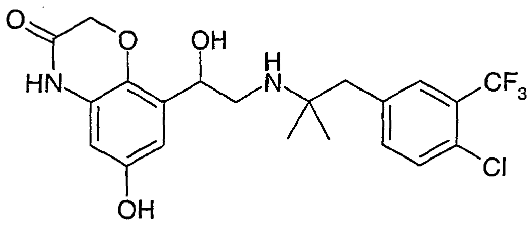

### a) 1-(4-Chlor-3-trifluormethyl-phenyl)-2-methyl-propan-1ol

Herstellung in Analogie zu dem für Beispiel 1a) beschriebenen Verfahren aus 2.3 mL (15 mmol) 5-Brom-2-chlorbenzotrifluorid und 1.3 mL (14 mmol) Isobutyraldehyd. Gelbes Öl. Ausbeute: 2.0 g (53%).

### b) 1-Chlor-4-(2-methyl-propenyl)-2-trifluormethyl-benzol

1.50 g (5.9 mmol) 1-(4-Chlor-3-trifluormethyl-phenyl)-2-methyl-propan-1ol und 0.50 g (2.6 mmol) p-Toluensulfonsäure-Monohydrat in 50 mL Toluol werden 3 Stunden am Wasserabscheider refluxiert. Nach Aufarbeitung in Analogie zu Beispiel 1b) wird ein braunes Öl erhalten. Ausbeute: 1.4 g; Massenspektrometrie: [M]⁺ = 234/6.

### c) N-(2-(4-Chlor-3-trifluormethyl-phenyl)-1,1-dimethyl-ethyl-formamid

Eine Ritter-Reaktion mit 1.30 g (5.5 mmol) 1-Chlor-4-(2-methyl-propenyl)-2-trifluormethyl-benzol nach dem für Beispiel 1c) beschriebenen Verfahren liefert die Zielverbindung in Form eines braunen Öls. Ausbeute: 1.50 g (97%); Massenspektrometrie: [M+H]⁺ = 280/2.

### d) 2-(4-Chlor-3-trifluormethyl-phenyl)-1,1-dimethyl-ethylamin

Darstellung in Analogie zu Beispiel 1d) aus 1.50 g (5.4 mmol) N-[2-(4-Chlor-3-trifluormethyl-phenyl)-1,1-dimethyl-ethyl-formamid. Braunes Öl. Ausbeute: 1.19 g (88%); Massenspektrometrie: [M+H]⁺ = 252/4.

### e) 6-Benzyloxy-8-{2-[2-(4-chlor-3-trifluormethyl-phenyl)-1,1-dimethyl-ethylamin]-1-hydroxy-ethyl}-4H-benzo[1,4]oxazin-3-on

357 mg (1 mmol) 6-Benzyloxy-8-(2-ethoxy-2-hydroxy-acetyl)-4H-benzo[1,4]oxazin-3-on und 252 mg (1 mmol) 2-(4-Chlor-3-trifluormethyl-phenyl)-1,1-dimethyl-ethylamin werden in Analogie zur Arbeitsvorschrift für Beispiel 2c) umgesetzt und aufgearbeitet. Die abschließende Reinigung erfolgt mittels Chromatographie. Beigefarbener Feststoff. Ausbeute: 316 mg (48%, Trifluoracetat); Massenspektrometrie: [M+H]⁺ = 549/51.

### f) 8-{2-[2-(4-Chlor-3-trifluormethyl-phenyl)-1,1-dimethyl-ethylamin]-1-hydroxy-ethyl}-6-hydroxy-4H-benzo[1,4]xazin-3-on

316 mg (0.48 mmol) 6-Benzyloxy-8-{2-[2-(4-chlor-3-trifluormethyl-phenyl)-1,1-dimethylethylamin]-1-hydroxy-ethyl}-4H-benzo[1,4]oxazin-3-on werden in 3 mL Dichlormethan gelöst und auf -40°C abgekühlt. Bei dieser Temperatur werden 1.4 mL einer 1 molaren Lösung von Bortribromid in Dichlormethan zugegeben. Nach 10 Minuten wird die Reaktion durch Zugabe von Dichlormethan und Wasser beendet und die Lösung über Kieselgur filtriert. Das Filtrat wird von Lösungsmitteln befreit und der Rückstand chromatographiert (Reverse Phase; Wasser/Acetonitril-Gradient mit 0.1 % Trifluoressigsäure). Rosafarbener Feststoff. Ausbeute: 49 mg (18%, Trifluoracetat); Massenspektroskopie [M+H]⁺ = 459/61.

### Beispiel 6: 8-{2-[1,1-Dimethyl-2-(2-trifluormethyl-phenyl)-ethylamino]1-hydroxyethyl -6-hydroxy-4H-benzo[1,4]oxazin-3-on

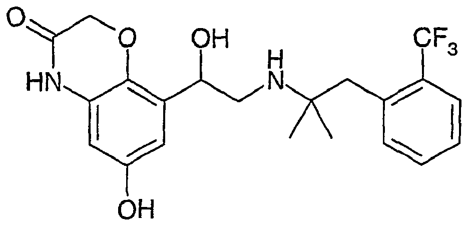

### a) 1-(4-Chlor-2-trifluormethyl-phenyl)-2-methyl-propan-1-ol

Aus 1.77 g (73 mmol) mit Iod aktiviertem Magnesium und 18.70 g (72 mol) 1-Brom-4-chlor-2-trifluormethyl-benzol gelöst in 150 mL Diethylether wird ein Grignard hergestellt. Zu dem Grignard-Reagenz werden bei Raumtemperatur 5.46 g (72 mmol) Isobutyraldehyd in 30 mL Diethylether getropft. Nach beendeter Zugabe läßt man die Reaktionsmischung 30 Minuten refluxieren und anschließend bei Raumtemperatur über Nacht rühren. Es wird mit Eiswasser versetzt und mit Salzsäure angesäuert. Die wässrige Phase wird abgetrennt und mit Diethylether extrahiert. Die organischen Phasen werden vereinigt, mit Natriumsulfat getrocknet und eingeengt. Der Rückstand wird an einer Kieselgelsäule chromatographiert (Petrolether/Ethylacetat = 9:1). 10.5 g Öl.

### b) 4-Chlor-1-(2-methyl-propenyl)-2-trifluormethyl-benzol

8.00 g (42 mmol) 1-(4-Chlor-2-trifluormethyl-phenyl)-2-methyl-propan-1-ol und 2.5 g p-Toluolsulfonsäure-Monohydrat in 200 mL Toluol werden über Nacht unter Rückfluß erhitzt, wobei entstehendes Wasser mit einen Wasserabscheider aufgefangen wird. Man läßt die Reaktionsmischung abkühlen, versetzt mit Wasser und trennt die organische Phase ab. Es wird mit Toluol extrahiert und die organischen Phasen werden vereinigt und eingeengt. Der Rückstand wird auf eine kurze Kieselgelsäule gegeben und mit Hexan eluiert. Farbloses Öl. Ausbeute: 6.1 g (63%); Massenspektroskopie [M]⁺ = 234/6.

### c) N-[2-(4-Chlor-2-trifluormethyl-phenyl)-1,1-dimethyl-ethyl]-formamid

Nach dem für Beispiel 1c) beschriebenen Verfahren werden 5.9 g (25 mmol) 4-Chlor-1-(2-methyl-propenyl)-2-trifluormethyl-benzol in einer Ritter-Reaktion umgesetzt und aufgearbeitet. Gelbes Öl. Ausbeute: 5.7 g (81%); Massenspektroskopie [M+H]⁺ = 280/2.

### d) 2-(4-Chlor-2-trifluormethyl-phenyl)-1,1-dimethyl-ethylamin

5.70 g (20 mmol) N-[2-(4-Chlor-2-trifluormethyl-phenyl)-1,1-dimethyl-ethyl]-formamid und 3. 00 g Kaliumhydroxid werden über Nacht bei 140°C in 20 mL Ethylenglycol gerührt. Die Reaktionsmischung wird mit Wasser versetzt und wiederholt mit Dichlormethan extrahiert. Die vereinigten organischen Phasen werden mit Wasser gewaschen, mit Natriumsulfat getrocknet und eingeengt. Der Rückstand wird an einer kurzen Säule mit Kieselgel filtriert (Laufmittel: Dichlormethan/Methanol/Ammoniak = 9:1:0.1). Ausbeute: 3.7 g (51%); Massenspektroskopie [M+H]⁺ = 252/4.

### e) 8-{2-[1,1-Dimethyl-2-(2-trifluormethyl-phenyl)-ethylamino]-1-hydroxy-ethyl}-6-hydroxy-4H-benzo[1,4]oxazin-3-on

357 mg (1 mmol) 6-Benzyloxy-8-(2-ethoxy-2-hydroxy-acetyl)-4H-benzo[1,4]oxazin-3-on und 252 mg (1 mmol) 2-(4-Chlor-2-trifluormethyl-phenyl)-1,1-dimethyl-ethylamin werden analog dem für Beispiel 2c) beschriebenen Verfahren umgesetzt und aufgearbeitet. Weißer Feststoff. Ausbeute: 165 mg (31%, Trifluoracetat); Massenspektroskopie [M+H]⁺ = 425.

### Beispiel 7: 8-{2-[2-(3,5-Bis-trifluormethyl-phenyl)-1,1-dimethyl-ethylamino]-1-hydroxyethyl}-6-hydroxy-4H-benzo[1,4]oxazin-3-on

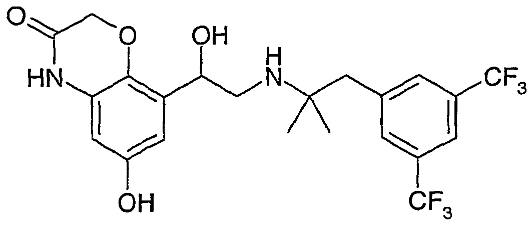

### a) 1-(2-Methyl-propenyl)-3,5-bis-trifluormethyl-benzol

15.88 g (36 mmol) Isopropyltriphenylphosphoniumiodid werden in 210 mL Diethylether/Tetrahydrofuran (1: 1) gelöst und auf -10°C abgekühlt. Bei dieser Temperatur werden 25 mL einer 1.6 molaren Lösung von Butyllithium in Hexan zugetropft. Anschließend wird 3 h bei Raumtemperatur gerührt und dann auf -50°C abgekühlt. 9.98 g (40 mmol) 3,5-Bis-trifluormethyl-benzaldehyd gelöst in 15 ml Tetrahydrofuran werden zugetropft und es wird über Nacht unter Erwärmung auf Raumtemperatur gerührt. Die Reaktionsmischung wird mit Wasser und Hexan versetzt, 30 Minuten gerührt und dann filtriert. Die organische Phase wird abgetrennt, wiederholt mit Wasser gewaschen, mit Natriumsulfat getrocknet und eingeengt. Der Rückstand wird mittels Chromatographie an einer kurzen Kieselgelsäule gereinigt (Hexan/Ethylacetat = 50:1). Gelbes Öl. Ausbeute: 4.50 g (42%); Massenspektroskopie [M]⁺ = 268.

### b) N-[2-(3,5-Bis-trifluormethyl-phenyl)-1,1-dimethyl-ethyl]-formamid

Die Zielverbindung wird aus der Umsetzung von 4.29 g (16 mmol) 1-(2-Methyl-propenyl)-3,5-bis-trifluormethyl-benzol nach dem für Beispiel 1c) beschriebenen Verfahren erhalten. Abweichend von dieser Vorschrift wird das Rohprodukt am Ende zusätzlich noch an einer kurzen Kieselgelsäule filtriert (Laufmittel: Dichlormethan/Methanol/Ammoniak = 9:1:0.1). Beigefarbener Feststoff. Ausbeute: 1.00 g (20%); Massenspektroskopie [M+H]⁺ = 314.

### c) 2-(3,5-Bis-trifluormethyl-phenyl)-1,1-dimethyl-ethylamin

Herstellung analog dem für Beispiel 1d) beschriebenen Verfahren aus 1.40 g (4.4 mmol) N-[2-(3,5-Bis-trifluormethyl-phenyl)-1,1-dimethyl-ethyl]-formamid. Braunes Öl. Ausbeute: 0.60 g (47%); Massenspektroskopie [M+H]⁺ = 286.

### d) 8-{2-[2-(3,5-Bis-trifluormethyl-phenyl)-1,1-dimethyl-ethylamino]-1-hydroxy-ethyl}-6-hydroxy-benzo[1,4]oxazin-3-on

Herstellung aus 357 mg (1 mmol) 6-Benzyloxy-8-(2-ethoxy-2-hydroxy-acetyl)-4H-benzo[1,4]oxazin-3-on und 285 mg (1 mmol) 2-(3,5-Bis-trifluormethyl-phenyl)-1,1-dimethyl-ethylamin in Analogie zu dem für Beispiel 2c) beschriebenen Verfahren. Abweichend wird die Hydrierung unter Verwendung von Raney-Nickel durchgeführt. Nach dem Absaugen des Katalysators wird das Filtrat eingeengt und der Rückstand an einer kurzen Kieselgelsäule filtriert (Laufmittel: Dichlormethan/Methanol = 9:1). Die produkthaltigen Fraktionen werden vom Lösungsmittel befreit und in Wasser/Acetonitril verrührt. Brauner Feststoff. Ausbeute: 7 mg (2%); Massenspektroskopie [M+H]⁺ = 493.

Die (R)- und (S)-Enantiomere der vorstehend genannten Ausführungsbeispiele können durch Auftrennung der Racemate in Analogie zu gängigen, im Stand der Technik bekannten Methoden erhalten werden.

Die Verbindungen der allgemeinen Formel **1** können allein oder in Kombination mit anderen erfindungsgemäßen Wirkstoffen der Formel **1** zur erfindungsgemäßen Anwendung gelangen. Gegebenenfalls können die Verbindungen der allgemeinen Formel **1** auch in Kombination mit weiteren pharmakologisch aktiven Wirkstoffen eingesetzt werden.

Es handelt sich hierbei insbesondere um Anticholinergika, gegebenenfalls andere Betamimetika, Antiallergika, PDE IV-Inhibitoren, PAF-Antagonisten, Leukotrien-Antagonisten, EGFR-Hemmer und Corticosteroiden sowie Wirkstoffkombinationen davon.

Als bevorzugte Beispiele für Anticholinergika sind zu nennen Ipratropium-, Oxitropium-und Tiotropiumsalze. Arzneimittelkombinationen, die neben den erfindungsgemäßen Verbindungen der Formel **1** vorstehend genannte Salze enthalten, enthalten bevorzugt solche Salze des Ipatropiums, Oxitropiums oder Tiotropiums in denen das Anion ausgewählt ist aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, gegebenenfalls in Form eines ihrer Solvate oder Hydrate.

Im Rahmen der vorliegenden Erfindung werden unter Corticosteroiden, die gegebenenfalls in Kombination mit der Verbindung der Formel **1** zum Einsatz gelangen können, Verbindungen verstanden, die ausgewählt sind aus der Gruppe bestehend aus Flunisolide, Beclomethasone, Triamcinolone, Budesonid, Fluticasone, Mometasone, Ciclesonide, Rofleponide und Dexametasone. Gegebenenfalls wird im Rahmen der vorliegenden Patentanmeldung statt der Bezeichnung Corticosteroide auch nur die Bezeichnung Steroide verwendet. Eine Bezugnahme auf Steroide schließt im Rahmen der vorliegenden Erfindung eine Bezugnahme auf Salze oder Derivate, die von den Steroiden gebildet werden können, mit ein. Als mögliche Salze oder Derivate werden beispielsweise genannt: Natriumsalze, Sulfobenzoate, Phosphate, Isonicotinate, Acetate, Propionate, Dihydrogenphosphate, Palmitate, Pivalate oder Furoate. Gegebenenfalls können die Corticosteroide auch in Form ihrer Hydrate vorliegen.

Im Rahmen der vorliegenden Erfindung werden unter Dopamin-Agonisten, die gegebenenfalls in Kombination mit der Verbindung der Formel **1** zum Einsatz gelangen können, Verbindungen verstanden, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan. Eine Bezugnahme auf die vorstehend genannten Dopamin-Agonisten schließt im Rahmen der vorliegenden Erfindung eine Bezugnahme auf deren gegebenenfalls existierende pharmakologisch verträgliche Säureadditionssalze und gegebenenfalls deren Hydrate ein. Unter den physiologisch verträglichen Säureadditionssalzen, die von den vorstehend genannten Dopaminagonisten gebildet werden können, werden beispielsweise pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure sind.

Als Beispiel für Antiallergika, die erfindungsgemäß mit der Verbindung der Formel 1 als Kombination zum Einsatz kommen können, seien genannt Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin. Eine Bezugnahme auf die vorstehend genannten Antiallergika schließt im Rahmen der vorliegenden Erfindung eine Bezugnahme auf deren gegebenenfalls existierende pharmakologisch verträgliche Säureadditionssalze ein.

Als Beispiel für PDE-IV-Inhibitoren, die erfindungsgemäß mit der Verbindung der Formel **1** als Kombination zum Einsatz kommen können, seien genannt Verbindungen, die ausgewählt sind aus der Gruppe bestehend aus Enprofylline, Roflumilast, Ariflo, Bay-198004, CP-325,366, BY343, D-4396 (Sch-351591), V-11294A und AWD-12-281. Eine Bezugnahme auf die vorstehend genannten PDE-IV-Inhibitoren schließt im Rahmen der vorliegenden Erfindung eine Bezugnahme auf deren gegebenenfalls existierende pharmakologisch verträgliche Säureadditionssalze ein. Unter den physiologisch verträglichen Säureadditionssalzen, die von den vorstehend genannten PDE-IV-Inhibitoren gebildet werden können, werden erfindungsgemäß pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure sind. Erfindungsgemäß bevorzugt sind in diesem Zusammenhang die Salze ausgewählt aus der Gruppe bestehend aus Acetat, Hydrochlorid, Hydrobromid, Sulfat, Phosphat und Methansulfonat.

Als Beispiel für EGFR-Hemmer, die erfindungsgemäß mit der Verbindung der Formel **1** als Kombination zum Einsatz kommen können, seien genannt, Verbindungen, die ausgewählt sind aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluor-phenyl)amino]-6-{[4-((R)-6-methyl-2-oxomorpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Ethinylphenyl)amino]-6,7-bis-(2-methoxy-ethoxy)-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxychinolin, 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-{2-[4-(2-oxomorpholin-4-yl)-piperidin-1-yl]-ethoxy}-7-methoxy-chinazolin, 4-[(3-Chlor-4-fluorphenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cyclohexan-1-yloxy)-7-methoxychinazolin, 4-[(3-Chlor-4-fluor-phenyl)amino]-6-(tetrahydropyran-3-yloxy)-7-methoxychinazolin, 4-[(3-Chlor-4-fluor-phenyl)amino]-6-{1-[(morpholin-4-yl)carbonyl]-piperidin-4-yloxy}-7-methoxy-chinazolin, 4-[(3-Chlor-4-fluor-phenyl)amino]-6-(piperidin-3-yloxy)-7-methoxy-chinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4.-fluor-phenyl)amino]-6-{trans-4-[(morpholin-4-yl)carbonylamino]-cyclohexan-1-yloxy}-7-methoxy-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-(trans-4-ethansulfonylamino-cyclohexan-1-yloxy)-7-methoxychinazolin, 4-[(3-Chlor-4-fluor-phenyl)amino]-6-(1-methansulfonyl-piperidin-4-yloxy)-7-(2-methoxy-ethoxy)-chinazolin, 4-[(3-Chlor-4-fluor-phenyl)amino]-6-[1-(2-methoxyacetyl)-piperidin-4-yloxy]-7-(2-methoxy-ethoxy)-chinazolin, 4-[(3-Ethinyl-phenyl)amino]-6-(tetrahydropyran-4-yloxy]-7-methoxy-chinazolin, 4-[(3-Chlor-4-fluor-phenyl)amino]-6-(cis-4-{N-[(piperidin-1-yl)carbonyl]-N-methyl-amino}-cyclohexan-1-yloxy)-7-methoxychinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Ethinylphenyl)amino]-6-(1-acetyl-piperidin-4-yloxy)-7-methoxy-chinazolin, 4-[(3-Ethinylphenyl)amino]-6-(1-methyl-piperidin-4-yloxy)-7-methoxy-chinazolin, 4-[(3-Ethinylphenyl)amino]-6-(1-methansulfonyl-piperidin-4-yloxy)-7-methoxy-chinazolin, 4-[(3-Chlor-4-fluor-phenyl)amino]-6-(1-methyl-piperidin-4-yloxy)-7(2-methoxy-ethoxy)-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluor-phenyl)amino]-6-(1-ethyl-piperidin-4-yloxy)-7-methoxy-chinazolin, und 4-[(3-Chlor-4-fluor-phenyl)amino]-6-{1-[(2-methoxyethyl)carbonyl]-piperidin-4-yloxy}-7-methoxychinazolin. Eine Bezugnahme auf die vorstehend genannten EGFR-Hemmer schließt im Rahmen der vorliegenden Erfindung eine Bezugnahme auf deren gegebenenfalls existierende pharmakologisch verträgliche Säureadditionssalze ein. Unter den physiologisch verträglichen Säureadditionssalzen, die von den vorstehend genannten EGFR-Hemmern gebildet werden können, werden erfindungsgemäß pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure sind. Erfindungsgemäß bevorzugt sind in diesem Zusammenhang die Salze ausgewählt aus der Gruppe bestehend aus Acetat, Hydrochlorid, Hydrobromid, Sulfat, Phosphat und Methansulfonat.

Geeignete Anwendungsformen zur Applikation der Verbindungen der Formel **1** sind beispielsweise Tabletten, Kapseln, Zäpfchen, Lösungen, Pulver etc. Der Anteil der pharmazeutisch wirksamen Verbindung(en) sollte jeweils im Bereich von 0,05 bis 90 Gew.-%, bevorzugt 0,1 bis 50 Gew.-% der Gesamtzusammensetzung liegen. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können. Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Lösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösungsmittel als Lösevermittler bzw. Hilflösungsmittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.
Als Hilfsstoffe seien beispielsweise Wasser, pharmazeutisch unbedenkliche organische Lösungsmittel, wie Paraffine (z.B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide), synthetische Gesteinsmehle (z.B. hochdisperse Kieselsäure und Silikate), Zucker (z.B. Rohr-, Milch- und Traubenzucker), Emulgiermittel (z.B. Lignin, Sufitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Bei der erfindungsgemäßen Applikation der Verbindungen der Formel **1** zur Therapie von COPD werden besonders bevorzugt inhalativ applizierbare Darreichungsförmen bzw. pharmazeutische Formulierungen eingesetzt. Als inhalierbare Darreichungsformen kommen Inhalationspulver, treibgashaltige Dosieraerosole oder treibgasfreie Inhalationslösungen in Betracht. Im Rahmen der vorliegenden Erfindung sind von dem Begriff treibgasfreie Inhalationslösungen auch Konzentrate oder sterile, gebrauchsfertige Inhalationslösungen umfaßt. Die im Rahmen der vorliegenden Erfindung einsetzbaren Darreichungsformen werden im nachfolgenden Teil der Beschreibung detailliert beschrieben.

Erfindungsgemäß einsetzbare Inhalationspulver können **1** entweder allein oder im Gemisch mit geeigneten physiologisch unbedenkliche Hilfsstoffen enthalten.
Sind die Wirkstoffe **1** im Gemisch mit physiologisch unbedenklichen Hilfsstoffen enthalten, können zur Darstellung dieser erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.
Die Hilfsstoffe weisen im Rahmen der erfindungsgemäßen Inhalationspulver eine maximale mittlere Teilchengröße von bis zu 250µm, bevorzugt zwischen 10 und 150µm, besonders bevorzugt zwischen 15 und 80 µm auf. Gegebenenfalls kann es sinnvoll erscheinen, den vorstehend genannten Hilfststoffen feinere Hilfsstofffraktionen mit einer mittleren Teilchengröße von 1 bis 9 µm beizumischen. Letztgenannte feinere Hilfsstoffe sind ebenfalls ausgewählt aus der vorstehend genannten Gruppe an einsetzbaren Hilfsstoffen. Schließlich wird zur Herstellung der erfindungsgemäßen Inhalationspulver mikronisierter Wirkstoff **1**, vorzugsweise mit einer mittleren Teilchengröße von 0,5 bis 10µm, besonders bevorzugt von 1 bis 5µm, der Hilfsstoffmischung beigemischt. Verfahren zur Herstellung der erfindungsgemäßen Inhalationspulver durch Mahlen und Mikronisieren sowie durch abschließendes Mischen der Bestandteile sind aus dem Stand der Technik bekannt.

Die erfindungsgemäßen Inhalationspulver können mittels aus dem Stand der Technik bekannten Inhalatoren appliziert werden.

Erfindungsgemäße treibgashaltige Inhalationsaerosole können **1** im Treibgas gelöst oder in dispergierter Form enthalten. Hierbei können **1** in getrennten Darreichungsformen oder in einer gemeinsamen Darreichungsform enthalten sein, wobei **1** entweder beide gelöst, beide dispergiert oder jeweils nur eine Komponente gelöst und die andere dispergiert enthalten sein können.
Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie fluorierten Derivaten des Methans, Ethans, Propans, Butans, Cyclopropans oder Cyclobutans. Die vorstehend genannten Treibgase können dabei allein oder in Mischungen derselben zur Verwendung kommen. Besonders bevorzugte Treibgase sind halogenierte Alkanderivate ausgewählt aus TG134a und TG227 und Mischungen derselben.

Die treibgashaltigen Inhalationsaerosole können ferner weitere Bestandteile wie Kosolventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

Die vorstehend genannten treibgashaltigen Inhalationaerosole können mittels im Stand der Technik bekannten Inhalatoren (MDIs = metered dose inhalers) appliziert werden.

Ferner kann die Applikation der erfindungsgemäßen Wirkstoffe **1** in Form von treibgasfreien Inhalationslösungen und Inhalationssuspensionen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Der relative Anteil an Ethanol gegenüber Wasser ist nicht begrenzt, bevorzugt liegt die maximale Grenze jedoch bei bis 70 Volumenprozent, insbesondere bei bis zu 60 Volumenprozent und besonders bevorzugt bei bis zu 30 Volumenprozent. Die restlichen Volumenprozente werden von Wasser aufgefüllt. Die **1** enthaltenden Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.
In diesen Formulierungen kann gegebenenfalls auf den Zusatz von Editinsäure (EDTA) oder einem der bekannten Salze davon, Natriumedetat, als Stabilisator oder Komplexbildner verzichtet werden. Andere Ausführungsformen beinhalten diese Verbindung(en). In einer solchen bevorzugten Ausführungsform liegt der Gehalt bezogen auf Natriumedetat unter 100 mg / 100 ml, bevorzugt unter 50 mg/ 100ml, besonders bevorzugt unter 20 mg/ 100ml. Generell sind solche Inhalationslösungen bevorzugt, in denen der Gehalt an Natriumedetat bei 0 bis 10mg/100ml liegt.
Den treibgasfreien Inhaltionslösungen können Co-Solventien und/oder weitere Hilfsstoffe zugesetzt werden. Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester. Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien.
Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.
Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration. Die vorstehend genannten Konservierungsstoffe sind vorzugsweise in Konzentrationen von bis zu 50mg/100ml, besonders bevorzugt zwischen 5 und 20 mg/100ml enthalten.
Bevorzugte Formulierungen enthalten außer dem Lösungsmittel Wasser und dem Wirkstoff **1** nur noch Benzalkoniumchlorid und Natriumedetat.
In einer anderen bevorzugten Ausführungsform wird auf Natriumedetat verzichtet.

Die Dosierung der erfindungsgemäßen Verbindungen ist naturgemäß stark von der Applikationsart und der zu therapierenden Erkrankung abhängig. Bei inhalativer Applikation zeichnen sich die Verbindungen der Formel **1** bereits bei Dosen im µg-Bereich durch eine hohe Wirksamkeit aus. Auch oberhalb des µg-Bereichs, lassen sich die Verbindungen der Formel **1** sinnvoll einsetzen. Die Dosierung kann dann beispielsweise auch im Grammbereich liegen.

Ein weiterer Aspekt der vorliegenden Erfindung betrifft die vorstehend genannten pharmazeutischen Formulierungen, gekennzeichnet durch einen Gehalt einer Verbindung der Formel **1,** als solche, besonders bevorzugt die vorstehend genannten inhalativ applizierbaren pharmazeutischen Formulierungen.

Die nachfolgenden Formulierungsbeipiele illustrieren die vorliegende Erfindung, ohne sie jedoch in ihrem Umfang zu beschränken:

### Pharmazeutische Formulierungsbeispiele

| A) | Tabletten | pro Tablette |
|---|---|---|
| | Wirkstoff **1** | 100 mg |
| | Milchzucker | 140 mg |
| | Maisstärke | 240 mg |
| | Polyvinylpyrrolidon | 15 mg |
| | Magnesiumstearat | 5 mg |
| | | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

| B) | Tabletten | pro Tablette |
|---|---|---|
| | Wirkstoff **1** | 80 mg |
| | Milchzucker | 55 mg |
| | Maisstärke | 190 mg |
| | Mikrokristalline Cellulose | 35 mg |
| | Polyvinylpyrrolidon | 15 mg |
| | Natrium-carboxymethylstärke | 23 mg |
| | Magnesiumstearat | 2 mg |
| | | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpreßt das Gemisch zu Tabletten geeigneter Größe.

| C) | Ampullenlösung | |
|---|---|---|
| | | |
| | Wirkstoff **1** | 50 mg |
| | Natriumchlorid | 50 mg |
| | Aqua pro inj. | 5 ml |

Der Wirkstoff wird bei Eigen-pH oder gegebenenfalls bei pH 5,5 bis 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

| D) | Dosieraerosol | |
|---|---|---|
| | | |
| | Wirkstoff **1** | 0,005 |
| | Sorbitantrioleat | 0,1 |
| | Monofluortrichlormethan und TG134a : TG227 2:1 | ad 100 |

Die Suspension wird in einen üblichen Aerosolbehälter mit Dosierventil gefüllt. Pro Betätigung werden vorzugsweise 50 µl Suspension abgegeben. Der Wirkstoff kann gewünschtenfalls auch höher dosiert werden (z.B. 0.02 Gew.-%).

| E) | Lösungen (in mg/100ml) | |
|---|---|---|
| | Wirkstoff **1** | 333.3 mg |
| | Benzalkoniumchlorid | 10.0 mg |
| | EDTA | 50.0 mg |
| | HCl (ln) | ad pH 3.4 |

Diese Lösung kann in üblicher Art und Weise hergestellt werden.

| F) | Inhalationspulver | |
|---|---|---|
| | Wirkstoff **1** | 12 µg |
| | Lactose Monohydrat | ad 25 mg |

Die Herstellung des Inhalationspulvers erfolgt in üblicher Art und Weise durch Mischen der einzelnen Bestandteile.

## Patentansprüche

1. Verbindungen der allgemeinen Formel **1** 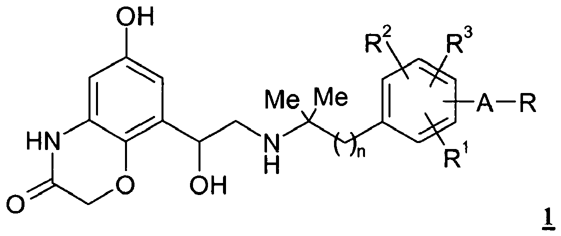 worin
n 1 oder 2;
A Sauerstoff oder eine Einfachbindung;
R -C₁-C₆-Alkyl, das ein- oder mehrfach durch ein oder mehrere Halogenatome substituiert ist;
R¹, R² und R³ gleich oder verschieden, Wasserstoff, C₁-C₆-Alkyl, Halogen-C₁-C₆-alkylen, OH, HO-C₁₋₆-alkylen, -O-C₁₋C₆-Alkyl, C₆-C₁₀-Aryl, C₆-C₁₀-Aryl-C₁-C₄-alkylen, C₆-C₁₀-Aryl-C₁-C₆-alkylen-O-, -COOH, -COOC₁-C₆-alkyl, -O-C₁-C₆-alkylen-COOH, -O-C₁-C₆-alkylen-COOC₁-C₆-alkyl, -NHSO₃H, -NHSO₂-C₁-C₆-alkyl, CN, NH₂, -NH-C₁-C₆-Alkyl, -N(C₁-C₆-Alkyl)₂, NO₂, -S-C₁-C₆-Alkyl, -SO₂-C₁-C₆-Alkyl, -SO-C₁-C₆-Alkyl, -O(CO)C₁-C₆-Alkyl, -COC₁-C₆-Alkyl, -NHCOC₁-C₆-Alkyl oder Halogen bedeuten.

2. Verbindungen der allgemeinen Formel **1** gemäß Anspruch 1, worin
n 1 oder 2;
A Sauerstoff oder eine Einfachbindung;
R C₁-C₄-Alkyl, das ein- oder mehrfach durch ein oder mehrere Halogenatome;
R¹, R² und R³ gleich oder verschieden, Wasserstoff, C₁-C₄-Alkyl, Halogen-C₁-C₄-alkylen, OH, HO-C₁-C₄-alkylen, -O-C₁-C₄-Alkyl, Phenyl, Phenyl-C₁-C₄-alkylen, Phenyl-C₁-C₄-alkylen-O-, -COOH, -COOC₁-C₄-alkyl, -O-C₁-C₄-alkylen-COOH, -O-C₁-C₄-alkylen-COOC₁-C₄-alkyl, -NHSO₂-C₁-C₄-alkyl, CN, NH₂, -NH-C₁-C₄-Alkyl, -N(C₁-C₄-Alkyl)₂, NO₂, -S-C₁-C₄-Alkyl, -SO₂-C₁-C₄-Alkyl, -SO-C₁-C₄-A[kyl, -O(CO)C₁-C₄-Alkyl, -COC₁-C₄-Alkyl, -NHCOC₁-C₄-Alkyl oder Halogen bedeuten.

3. Verbindungen der allgemeinen Formel **1** gemäß Anspruch 1 oder 2, worin
n 1 oder 2,
A Sauerstoff oder eine Einfachbindung;
R ein Methyl- oder Ethylrest, der ein-, zwei- oder dreifach durch ein oder mehrere Halogenatome ausgewählt aus Fluor oder Chlor substituiert ist;
R¹ und R² gleich oder verschieden, Wasserstoff, Halogen, C₁-C₄-Alkyl oder -O-C₁-C₄-Alkyl;
R³ Wasserstoff, C₁-C₄-Alkyl, OH, Halogen, -O-C₁-C₄-Alkyl, Halogen-C₁-C₄-alkylen, -O-C₁-C₄-Alkylen-COOH, -O-C₁-C₄-Alkylen-CO-O-C₁-C₄-Alkyl bedeuten.

4. Verbindungen der allgemeinen Formel 1 gemäß einem der Ansprüche 1 bis 3, worin
n 1;
A Sauerstoff oder eine Einfachbindung;
R -CH₂-CH₂Cl, -CH₂CCl₃, -CHCl-CCl₃, -CCl₃, -CH₂Cl, -CHCl₂, -CH₂CF₃, -CHF-CF₃, -CF₃, -CH₂F, -CH₂-CH₂F oder -CHF₂;
R¹ und R² gleich oder verschieden, Wasserstoff, Fluor, Chlor, Methyl oder Methoxy;
R³ Wasserstoff, C₁-C₄-Alkyl, OH, Fluor, Chlor, Brom, -O-C₁-C₄-Alkyl, -CF₃, -O-C₁-C₄-Alkylen-COOH, -O-C₁-C₄-Alkylen-CO-O-C₁-C₄-Alkyl, bedeuten.

5. Verbindungen der allgemeinen Formel **1** gemäß einem der Ansprüche 1 bis 4, worin
n 1;
A Sauerstoff oder eine Einfachbindung;
R -CH₂CF₃, -CHF-CF₃, -CF₃, -CH₂F, -CH₂-CH₂F oder -CHF₂;
R¹ und R² gleich oder verschieden Wasserstoff, Fluor, Chlor, Methyl oder Ethyl;
R³ Wasserstoff, Fluor, Chlor, Methyl, Ethyl, OH, Methoxy, Ethoxy, -CF₃, -O-CH₂-COOH, -O-CH₂-COOMethyl oder -O-CH₂-COOEthyl, bedeuten.

6. Verbindungen der allgemeinen Formel **1** nach einem der Ansprüche 1 bis 5, worin die Verbindungen der Formel **1** in Form der einzelnen optischen Isomeren, Mischungen der einzelnen Enantiomeren oder Racemate.

7. Verbindungen der allgemeinen Formel **1** nach einem der Ansprüche 1 bis 5, worin die Verbindungen der Formel **1** in Form der enantiomerenreinen Verbindungen vorliegen.

8. Verbindungen der allgemeinen Formel **1** gemäß einem der Ansprüche 1 bis 7, worin die Verbindungen der Formel **1** in Form ihrer Säureadditionssalze mit pharmakologisch unbedenklichen Säuren sowie gegebenenfalls in Form der Solvate und/oder Hydrate vorliegen.

9. Arzneimittel enthaltend Verbindungen der allgemeinen Formel **1** gemäß einem der Ansprüche 1 bis 8.

10. Verwendung der Verbindungen der allgemeinen Formel **1** gemäß einem der Ansprüche 1 bis 8, zur Herstellung eines Arzneimitels zur Behandlung von entzündlichen und obstruktiven Atemwegserkrankungen, insbesondere von Asthma oder COPD.

11. Pharmazeutischen Formulierung, **gekennzeichnet durch** einen Gehalt einer Verbindung der Formel **1** gemäß einem der Ansprüche 1 bis 8.

12. Inhaltiv applizierbare pharmazeutische Formulierung, **gekennzeichnet durch** einen Gehalt einer Verbindung der Formel **1** gemäß einem Ansprüche 1 bis 8.

13. Inhalativ applizierbare pharmazeutische Formulierung nach Anspruch 12, **dadurch gekennzeichnet, daß** sie ausgewählt ist aus der Gruppe bestehend aus Inhalationspulvern, treibgashaltige Dosieraerosole und treibgasfreie Inhalationslösungen.

## Claims

1. Compounds of general formula **1** 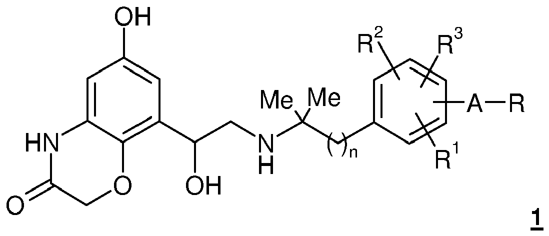 wherein
n denotes 1 or 2;
A denotes oxygen or a single bond;
R denotes -C₁-C₆-alkyl, which is mono- or polysubstituted by one or more halogen atoms;
R¹, R² and R³ which may be identical or different denote hydrogen, C₁-C₆-alkyl, halogen-C₁-C₆-alkylene, OH, HO-C₁₋₆-alkylene, -O-C₁-C₆-alkyl, C₆-C₁₀-aryl, C₆-C₁₀-aryl-C₁-C₄-alkylene, C₆-C₁₀-aryl-C₁-C₆-alkylene-O, -COOH, -COOC₁-C₆-alkyl, -O-C₁-C₆-alkylene-COOH, -O-C₁-C₆-alkylene-COOC₁-C₆-alkyl, -NHSO₃H, -NHSO₂-C₁-C₆-alkyl, CN, NH₂, -NH-C₁-C₆-alkyl, -N(C₁-C₆-alkyl)₂, NO₂, -S-C₁-C₆-alkyl, -SO₂-C₁-C₆-alkyl, -SO-C₁-C₆-alkyl, -O(CO)C₁-C₆-alkyl, -COC₁-C₆-alkyl, -NHCOC₁-C₆-alkyl or halogen.

2. Compounds of general formula **1** according to claim 1, wherein
n denotes 1 or 2;
A denotes oxygen or a single bond;
R denotes C₁-C₄-alkyl, which is mono- or polysubstituted by one or more halogen atoms;
R¹, R² and R³ which may be identical or different denote hydrogen, C₁-C₄-alkyl, halogen-C₁-C₄-alkylene, OH, HO-C₁-C₄-alkylene, -O-C₁-C₄-alkyl, phenyl, phenyl-C₁-C₄-alkylene, phenyl-C₁-C₄-alkylene-O, -COOH, -COOC₁-C₄-alkyl, -O-C₁-C₄-alkylene-COOH, -O-C₁-C₄-alkylene-COOC₁-C₄-alkyl, -NHSO₂-C₁-C₄-alkyl, CN, NH₂, -NH-C₁-C₄-alkyl, -N(C₁-C₄-alkyl)₂, NO₂, -S-C₁-C₄-alkyl, -SO₂-C₁-C₄-alkyl, -SO-C₁-C₄-alkyl, -O(CO)C₁-C₄-alkyl, -COC₁-C₄-alkyl, -NHCOC₁-C₄-alkyl or halogen.

3. Compounds of general formula **1** according to claim 1 or 2, wherein
n denotes 1 or 2,
A denotes oxygen or a single bond;
R denotes a methyl or ethyl group which is mono-, di- or trisubstituted by one or more halogen atoms selected from fluorine or chlorine;
R¹ and R² which may be identical or different denote hydrogen, halogen, C₁-C₄-alkyl or -O-C₁-C₄-alkyl;
R³ denotes hydrogen, C₁-C₄-alkyl, OH, halogen, -O-C₁-C₄-alkyl, halogen-C₁-C₄-alkylene, -O-C₁-C₄-alkylene-COOH, -O-C₁-C₄-alkylene-CO-O-C₁-C₄-alkyl.

4. Compounds of general formula **1** according to one of claims 1 to 3, wherein
n denotes 1;
A denotes oxygen or a single bond;
R denotes -CH₂-CH₂Cl, -CH₂CCl₃, -CHCl-CCl₃, -CCl₃, -CH₂Cl, -CHCl₂, -CH₂CF₃, -CHF-CF₃, -CF₃, -CH₂F, -CH₂-CH₂F or -CHF₂;
R¹ and R² which may be identical or different denote hydrogen, fluorine, chlorine, methyl or methoxy;
R³ denotes hydrogen, C₁-C₄-alkyl, OH, fluorine, chlorine, bromine, -O-C₁-C₄-alkyl, -CF₃, -O-C₁-C₄-alkylene-COOH, -O-C₁-C₄-alkylene-CO-O-C₁-C₄-alkyl.

5. Compounds of general formula **1** according to one of claims 1 to 4, wherein
n denotes 1;
A denotes oxygen or a single bond;
R denotes -CH₂CF₃, -CHF-CF₃, -CF₃, -CH₂F, -CH₂-CH₂F or -CHF₂;
R¹ and R² which may be identical or different denote hydrogen, fluorine, chlorine, methyl or ethyl;
R³ denotes hydrogen, fluorine, chlorine, methyl, ethyl, OH, methoxy, ethoxy, -CF₃, -O-CH₂-COOH, -O-CH₂-COOmethyl or -O-CH₂-COOethyl.

6. Compounds of general formula **1** according to one of claims 1 to 5, wherein the compounds of formula **1** are in the form of the individual optical isomers, mixtures of the individual enantiomers or racemates.

7. Compounds of general formula **1** according to one of claims 1 to 5, wherein the compounds of formula **1** are in the form of the enantiomerically pure compounds.

8. Compounds of general formula **1** according to one of claims 1 to 7, wherein the compounds of formula **1** are in the form of the acid addition salts thereof with pharmacologically acceptable acids and optionally in the form of the solvates and/or hydrates thereof.

9. Medicaments containing compounds of general formula **1** according to one of claims 1 to 8.

10. Use of the compounds of general formula **1** according to one of claims 1 to 8, for preparing a medicament for the treatment of inflammatory and obstructive respiratory complaints, particularly asthma or COPD.

11. Pharmaceutical formulation, **characterised in that** it contains a compound of formula **1** according to one of claims 1 to 8.

12. Pharmaceutical formulation administered by inhalation, **characterised in that** it contains a compound of formula **1** according to one of claims 1 to 8.

13. Pharmaceutical formulation administered by inhalation according to claim 12, **characterised in that** it is selected from the group comprising inhalable powders, propellant-driven metered-dose aerosols and propellant-free inhalable solutions.

## Revendications

1. Composé de formule générale 1 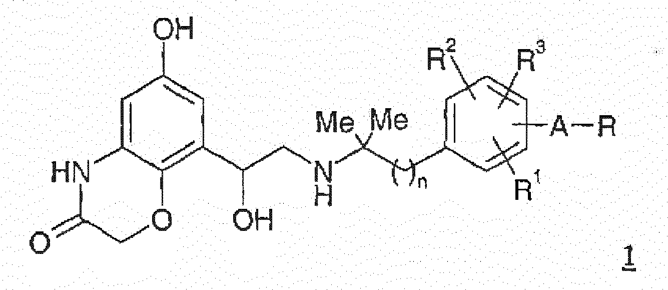 dans laquelle
n est 1 ou 2 ;
A désigne un atome d'oxygène ou une liaison simple ;
R désigne un groupe alkyle en C₁ à C₆ qui est mono- ou multi-substitué par un ou plusieurs atomes d'halogène ;
R¹, R² et R³, identiques ou différentes, désignent un atome d'hydrogène, un groupe alkyle en C₁ à C₆, halogéno-alkylène en C₁ à C₆, OH, HO-alkylène en C₁ à C₆, -O-alkyle en C₁ à C₆, aryle en C₆ à C₁₀, (aryle en C₆ à C₁₀) - (alkylène en C₁ à C₄), (aryle en C₆ à C₁₀) - (alkylène en C₁ à C₆) -O-, -COOH, -COO-(alkyle en C₁ à C₆), -O-(alkylène en C₁ à C₆)-COOH, -O-(alkylène en C₁ à C₆)-COO-(alkyle en C₁ à C₆) , -NHSO₃H, -NHSO₂-alkyle en C₁ à C₆, CN, NH₂, -NH-alkyle en C₁ à C₆, -N(alkyle en C₁ à C₆)₂, NO₂, -S-alkyle en C₁ à C₆, -SO₂-alkyle en C₁ à C₆, -SO-alkyle en C₁ à C₆, -O(CO)(alkyle en C₁ à C₆) , -CO-(alkyle en C₁ à C₆), -NHCO-(alkyle en C₁ à C₆) ou un atome d'halogène.

2. Composés de formule générale 1 selon la revendication 1, dans laquelle
n est 1 ou 2 ;
A désigne un atome d'oxygène ou une liaison simple ;
R désigne un groupe alkyle en C₁ à C₄ qui est mono- ou multi-substitué par un ou plusieurs atomes d'halogène ;
R¹, R² et R³, identiques ou différents, désignent un atome d'hydrogène, un groupe alkyle en C₁ à C₄, halogéno-alkylène en C₁ à C₄, OH, HO-alkylène en C₁ à C₄, -O-alkyle en C₁à C₄, phényle, phényl-(alkylène en C₁à C₄), phényl-(alkylène en C₁ à C₄)-O, -COOH, -COO-(alkyle en C₁ à C₄), -O-(alkylène en C₁ à C₄)-COOH, -O-(alkylène en C₁ à C₄)-COO-(alkyle en C₁ à C₄) , -NHSO-alkyle en C₁ à C₄, CN, NH₂, -NH-alkyle en C₁ à C₄, -N(alkyle en C₁ à C₄)₂, NO, -S-alkyle en C₁ à C₄, -SO₂-alkyle en C₁ à C₄, -SO-alkyle en C₁ à C₄, -O(CO)-(alkyle en C₁ à C₄), -CO-(alkyle en C₁ à C₄), -NHCO-(alkyle en C₁ à C₄) ou un atome d'halogène.

3. Composés de formule générale 1 selon la revendication 1 ou 2, dans laquelle
n est 1 ou 2 ;
A désigne un atome d'oxygène ou une liaison simple ; R désigne un radical méthyle ou éthyle qui est mono-, bi- ou trisubstitué par un ou plusieurs atomes d'halogène choisis parmi le fluor ou le chlore ;
R¹ et R², identiques ou différents, désignent un atome d'hydrogène, un atome d'halogène, un groupe alkyle en C₁ à C₄ ou -O-alkyle en C₁ à C₄ ;
R³ désigne un atome d'hydrogène, un groupe alkyle en C₁ à C₄, OH, un atome d'halogène, un groupe - O-alkyle en C₁ à C₄, hélogéno-alkylène en C₁ à C₄, -O-(alkylène en C₁ à C₄)-COOH, -O-(alkyléne en C₁ à C₄)-CO-O-(alkyle en C₁ à C₄).

4. Composés de formule générale 1 selon l'une des revendications 1 à 3, dans laquelle n est 1 ;
A désigne un atome d'oxygène ou une liaison simple ;
R désigne -CH₂-CH₂Cl, -CH₂CCl₃, -CHCl-CCl₃, -CCl₃, -CH2Cl, -CHCl₂, -CH₂CF₃, -CHF-CF₃, -CF₃, -CH₂F, -CH₂-CH₂F ou CHF₂ ;
R¹ et R², identiques ou différents, désignent un atome d'hydrogène, de fluor, de chlore, un groupe méthyle ou méthoxy ;
R³ désigne un atome d'hydrogène, un groupe alkyle en C₁ à C₄, OH, un atome de fluor, de chlore, de brome, un groupe -O-alkyle en C₁ à C₄, -CF₃, -O-(alkylène en C₁ à C₄)-COOH, -O-(alkylène en C₁ à C4-CO-O-(alkyle en C₁ à C₄).

5. Composés de formule générale 1 selon l'une des revendications 1 à 4, dans laquelle
n est 1 ;
A désigne un atome d'oxygène ou une liaison simple R désigne -CH₂CF₃, -CHF-CF₃, -CF₃, -CH₂F, -CH₂-CH₂F ou -CHF₂ ;
R¹ et R² identiques ou différents, désignent un atome d'hydrogène, de fluor, de chlore, un groupe méthyle ou éthyle ;
R³ désigne un atome d'hydrogène, de fluor, de chlore, un groupe méthyle, éthyle, OH, méthoxy, éthoxy, -CF₃, -O-CH2-COOH, -O-CH₂-COO-méthyle ou -O-CH₂-COO-éthyle.

6. Composés de formule générale 1 selon l'une des revendications 1 à 5, dans laquelle les composés de formule 1 se présentent sous la forme des isomères optiques individuels, de mélanges d'énantiomères individuels ou de racémates.

7. Composés de formule générale 1 selon l'une des revendications 1 à 5, dans laquelle les composés de formule 1 se présentent sous la forme de composés énantiopurs.

8. Composés de formule générale 1 selon l'une des revendications 1 à 7, dans laquelle les composés de formule 1 se présentent sous la forme de leurs sels d'addition acide avec des acides pharmacologiquement acceptables et éventuellement sous la forme de solvates et/ou d'hydrates.

9. Médicament contenant des composés de formule générale 1 selon l'une des revendications 1 à 8.

10. Utilisation des composés de formule générale 1 selon l'une des revendications 1 à 8, pour la production d'un médicament destiné au traitement de maladies inflammatoires et obstructives des voies respiratoires, en particulier de l'asthme ou de la BPCO.

11. Formulation pharmaceutique, **caractérisée par** une teneur en composé de formule 1 selon l'une des revendications 1 à 8.

12. Formulation pharmaceutique applicable par inhalation, **caractérisée par** une teneur en composé de formule 1 selon l'une des revendications 1 à 8.

13. Formulation pharmaceutique applicable par inhalation selon la revendication 12, **caractérisée en ce qu'**elle est choisie dans le groupe comprenant les poudres pour inhalation, les aérosols doseurs contenant un gaz propulseur et les solutions pour inhalation sans gaz propulseur.
